## Europäisches Patentamt
## European Patent Office
## Office européen des brevets

(19)

(11) Numéro de publication: **0 159 240**
**B1**

# FASCICULE DE BREVET EUROPÉEN

(12)

(45) Date de publication du fascicule du brevet:
25.11.87

(21) Numéro de dépôt: 85400537.8

(22) Date de dépôt: 21.03.85

(51) Int. Cl.⁴: **C 07 C 45/30,** C 07 C 45/64,
C 07 C 45/80, C 07 C 49/687,
C 07 C 49/713

(54) Procédé de préparation de l'hydroxy-4 triméthyl-2,4,6 cyclohexadiène-2,5 one.

(30) Priorité: 22.03.84 FR 8404442

(43) Date de publication de la demande:
23.10.85 Bulletin 85/43

(45) Mention de la délivrance du brevet:
25.11.87 Bulletin 87/48

(84) Etats contractants désignés:
AT BE CH DE FR GB IT LI LU NL SE

(56) Documents cité:
EP-A-0 018 606
EP-A-0 064 158

ACTA CHEMICA SCANDINAVICA, vol. B 36, no. 10, 1982, pages 675-683, Acta Chemica Scandinavica; K.-E. BERGQUIST et al.: "Electrophilic chlorination of 4-methylphenols with molecular chlorine. Synthesis of dimethoxy aromtics by methanolysis of 4-chloro-4-methylcyclohexa-2,5-dienones"

Le dossier contient des informations techniques présentées postérieurement au dépôt de la demande et ne figurant pas dans le présent fascicule.

(73) Titulaire: RHONE- POULENC SANTE, Les Miroirs 18 Avenue d'Alsace, F-92400 Courbevoie Cédex (FR)

(72) Inventeur: Costantini, Michel, 9 place Aristide Briand, F-69003 Lyon (FR)
Inventeur: Igersheim, Françoise, 21 rue Florian, F-69100 Villeurbanne (FR)
Inventeur: Krumenacker, Léon, 2 allée du Bois-Rond, F-69380 Sérézin du Rhône (FR)

(74) Mandataire: Pilard, Jacques, RHONE- POULENC RECHERCHES Service Brevets Pharma 25, Quai Paul Doumer, F-92408 Courbevoie Cedex (FR)

EP 0 159 240 B1

## Description

La présente invention concerne un nouveau procédé de préparation de l'hydroxy-4 triméthyl-2,4,6 cyclohexadiène-2,5 one qui constitue un intermédiaire pour la synthèse de la triméthylhydroquinone (TMHQ) qui est elle-même un précurseur de la vitamine E.

Il est connu de préparer l'hydroxy-4 triméthyl-2,4,6 cyclohexadiène-2,5 one par oxydation du triméthyl-2,4,6 phénol au moyen par exemple d'un peracide ou d'oxygène moléculaire en milieu basique. Cependant l'oxydation à l'air sous une pression voisine de 100 bars présente des difficultés de réalisation technique liées à des problèmes importants de sécurité.

Selon le procédé décrit par A. Nilson et coll., Tetrahedron Letters, 1107 (1975), une hydroxy-4 cyclohexadiène-2,5 one peut être obtenue par solvolyse d'une chloro-4 cyclohexadiène-2,5 one en présence d'eau et d'un sel d'argent. La chloro-4 cyclohexadiène-2,5 one peut être obtenue par action d'un agent de chloration tel que le chlore gazeux dans un solvant organique tel que le dichlorométhane ou le diméthylformamide selon K.E. Bergquist et coll., Acta Chimica Scandinavia, B 36, 675 (1982) ou par action du chlore dans l'anhydride acétique selon A. Fischer et G.N. Henderson, Can. J. Chem., 57, 552 (1979) sur un alcoyl-4 phénol.

Cependant l'action du chlore sur le triméthyl-2,4,6 phénol ou bien conduit à un mélange de chloro-4 triméthyl-2,4,6 cyclohexadiène-2,5 one et de chloro-3 triméthyl-2,4,6 phénol ou bien nécessite, pour obtenir de bons rendements l'emploi d'un solvant coûteux tel que l'anhydride acétique.

Par ailleurs, la transformation connue de la chloro-4 triméthyl-2,4,6 cyclohexadiène-2,5 one nécessite l'emploi d'un sel d'argent ce qui rend le procédé difficilement utilisable industriellement.

Pour améliorer la préparation de l'hydroxy-4 triméthyl-2,4,6 cyclohexadiène-2,5 one, il est possible de faire réagir sur le triméthyl-2,4,6 phénol un agent d'halogénation dans un solvant organique inerte à l'halogénation, en présence d'une base choisie parmi les bases minérales (soude, potasse, lithine, carbonate et bicarbonate de sodium et potassium, chaux, carbonate de calcium, acétate de sodium ou de potassium) ou organiques (triéthylamine, pyridines, pyridines substituées, diméthylformamide, anhydride acétique) puis d'effectuer l'hydrolyse par agitation dans l'eau éventuellement en présence d'une base.

Il a maintenant été trouvé, et c'est ce qui fait l'objet de la présente invention, que le procédé de préparation de l'hydroxy-4 triméthyl-2,4,6 cyclohexadiène-2,5 one pouvait encore être amélioré en effectuant la chloration du triméthyl-2,4,6 phénol dans un solvant organique convenable, en l'absence d'une base, et en éliminant l'acide chlorhydrique formé au fur et à mesure de sa formation par tout moyen physique approprié par exemple par entraînement par un courant de gaz inerte ou par distillation partielle du solvant, puis en agitant le mélange réactionnel avec de l'eau contenant éventuellement une base minérale.

Comme solvants organiques convenables peuvent être cités les hydrocarbures polychlorés tel que le chlorure de méthylène ou le tétrachlorure de carbone, les hydrocarbures saturés tels que les alcanes ou les cycloalcanes comme le pentane, l'hexane ou le cyclohexane, les hydrocarbures aromatiques comme le benzène, le toluène, les hydrocarbures aromatiques chlorés comme le chlorobenzène, les éthers comme l'éther diéthylique, l'éther diisopropylique, le méthyltertiobutyléther, le tétrahydrofuranne ou le dioxanne, les esters comme l'acétate d'éthyle ou les nitriles comme l'acétonitrile.

La concentration en triméthyl-2,4,6 phénol dans le solvant peut varier dans de larges limites. Elle est généralement comprise entre 0,1 mole/litre et la saturation.

Généralement, on utilise un rapport molaire agent d'halogénation/triméthyl-2,4,6 phénol compris entre 0,5 et 2 et, de préférence, entre 0,8 et 1,2.

La chloration peut être effectuée en opérant ou non avec un volume constant de mélange réactionnel. En opérant à volume constant, le volume de solvant entraîné par le courant gazeux ou par distillation est compensé par l'addition en continu dans le réacteur du même volume de solvant propre.

La chloration est généralement effectuée à une température comprise entre -20 et +100°C. Il est avantageux d'opérer à la température d'ébullition du solvant, la température pouvant être réglée en contrôlant la pression au sein du réacteur.

L'hydrolyse est généralement effectuée en ajoutant de l'eau au mélange réactionnel de telle manière que le rapport solvant/eau soit compris entre 0,1 et 10 et de préférence entre 0,5 et 2.

L'hydrolyse est effectuée généralement à une température comprise entre -10°C et la température d'ébullition du mélange réactionnel et de préférence entre 20 et 60°C.

L'hydrolyse peut être effectuée en présence ou en absence d'une base minérale telle que le bicarbonate de sodium.

Lorsque l'hydrolyse est effectuée en présence d'une base minérale, la quantité de base utilisée est dans un rapport molaire compris entre 0,1 et 2 par rapport au triméthyl-2,4,6 phénol mis en oeuvre et de préférence entre 0,5 et 1,5.

L'hydroxy-4 triméthyl-2,4,6 cyclohexadiène-2,5 one peut être isolée selon les méthodes habituelles d'extraction. Elle peut en particulier être isolée par extraction de la phase organique contenant l'hydroxy-4 triméthyl-2,4,6 cyclohexadiène-2,5 one par l'eau suivie de l'évaporation de la phase aqueuse après décantation ou par extraction de la solution aqueuse par un solvant organique non miscible qu'il suffit ensuite d'évaporer.

L'hydroxy-4 triméthyl-2,4,6 cyclohexadiène-2,5 one peut être transformée en triméthylhydroquinone dans les conditions décrites dans le brevet français FR 73 33374 publié sous le numéro 2 200 225 c'est-à-dire par chauffage à une température d'au moins 100° C dans un milieu liquide non acide tel que l'eau ou le méthanol aqueux.

Les exemples suivants, donnés à titre non limitatif, montrent comment l'invention peut être mise en pratique.

## Exemple 1

Dans un réacteur cylindrique comportant une agitation centrale, un tube plongeant d'arrivée de gaz (muni, en son extrémité, d'un verre fritté), un tube de sortie relié à un flacon-laveur contenant une solution aqueuse normale de soude, un thermomètre, une ampoule de coulée et un système de régulation thermique, on charge:

- triméthyl-2,4,6 phénol     13,6 g (100 m.moles)
- chlorure de méthylène     200 cm$^3$

On agite à une vitesse de 1000 tours/minute. Le mélange réactionnel est chauffé vers 35° C. On fait alors passer un mélange de chlore et d'azote (chlore/azote = 1/3 en volume) au débit de 20 litres/heure environ. La quantité de chlore introduite est mesurée par pesée et correspond à 8 g (112,6 m.moles). Les gaz effluents sont lavés par la solution de soude contenue dans le flacon-laveur. Le volume est maintenu constant par addition continue de chlorure de méthylène en quantité égale à celle entraînée par le courant gazeux. La durée de chloration est de 30 minutes.

On remplace alors le tube de sortie des gaz par un réfrigérant ascendant et on ajoute rapidement 200 cm$^3$ d'eau contenant 8,5 g (102 m.moles) de bicarbonate de sodium. On poursuit ensuite l'agitation pendant 3 heures à 38° C.

Après refroidissement et décantation, la phase aqueuse est extraite par 3 fois 50 cm$^3$ de chlorure de méthylène. Les phases organiques sont réunies.

Par chromatographie gaz-liquide, on dose:

- triméthyl-2,4,6 phénol     0,041 g (0,3 m.mole)
- hydroxy-4 triméthyl-2,4,6 cyclohexadiène-2,5 one     12,4 g (21,75 m.moles)

Le taux de transformation du triméthyl-2,4,6 phénol est de 99,7 %.
Le rendement en hydroxy-4 trimethyl-2,4,6 cyclohexadiène-2,5 one est de 82 %.

## Exemple 2

On opère comme dans l'exemple 1, en introduisant 7,1 g de chlore (100 m.moles) et en remplaçant l'azote par de l'argon.

On obtient les résultats suivants:

- triméthyl-2,4,6 phénol     2,40 g (17,7 m.moles)
- hydroxy-4 triméthyl-2,4,6 cyclohexadiène-2,5 one     10,2 g (67,1 m.moles)

Le taux de transformation du triméthyl-2,4,6 phénol est de 82,3 %.
Le rendement en hydroxy-4 triméthyl-2,4,6 cyclohexadiène-2,5 one est de 81,6 %.

## Exemple 3

On utilise le même réacteur que celui décrit dans l'exemple 1 dans lequel le tube de sortie est relié à un système permettant la distillation du solvant au cours de la chloration.
On charge:

- triméthyl-2,4,6 phénol     13,6 g (100 m.moles)
- chlorure de méthylène     200 cm$^3$

On agite à une vitesse de 1000 tours/minute. Le mélange réactionnel est chauffé au reflux (40° C). On fait alors passer un courant de chlore gazeux pendant 40 minutes. La quantité totale de chlore introduite est mesurée par pesée et correspond à 7,7 g (108 m.moles). En même temps que l'on fait passer le chlore, on distille le chlorure de méthylène tout en maintenant constant le volume réactionnel par addition continue de chlorure de méthylène. La quantité totale de chlorure de méthylène distillé est égale à 350 m$^3$.
On opère ensuite comme dans l'exemple 1.
Par chromatographie gaz-liquide, on dose:

- triméthyl-2,4,6 phénol     1,8 g (13,3 m.moles)
- hydroxy-4 triméthyl-2,4,6 cyclohexadiène-2,5 one     10,6 g (69,6 m.moles)

Le taux de transformation du triméthyl-2,4,6 phénol est de 86,7 %.
Le rendement en hydroxy-4 triméthyl-2,4,6 cyclohexadiène-2,5 one est de 80 %.

## Exemple 4

On opère comme dans l'exemple 1 mais en utilisant 7,1 g de chlore (100 m.moles) et en l'absence de bicarbonate de sodium.
Par chromatographie gaz-liquide, on dose:

- triméthyl-2,4,6 phénol     2,6 g (19,2 m.moles)
- hydroxy-4 triméthyl-2,4,6 cyclohexadiène-2,5 one     9,8 g (64,4 m.moles)

Le taux de transformation du triméthyl-2,4,6 phénol est de 80,8 %.
Le rendement en hydroxy-4 triméthyl-2,4,6 cyclohexadiène-2,5 one est de 79,7 %.

**Exemple 5**

On opère comme dans l'exemple 3 mais en effectuant la chloration en 1 heure 05 minutes et en distillant 90 cm³ de chlorure de méthylène.

Après dosage par chromatographie gaz-liquide, on obtient les résultats suivant:

- triméthyl-2,4,6 phénol .... 6,6 m.moles
- hydroxy-4 triméthyl-2,4,6 cyclohexadiène-2,5 one ... 71,0 m.moles

Le taux de transformation du triméthyl-2,4,6 phénol est de 93,3 %.

Le rendement en hydroxy-4 triméthyl-2,4,6 cyclohexadiène-2,5 one est de 76 %.

**Example 6**

On opère dans le même appareil que dans l'exemple 3. On charge:

- triméthyl-2,4,6 phénol    13,6 g (100 m.moles)
- méthyltertiobutyléther    200 cm³

On agite à la vitesse de 1000 tours/minute. Le mélange réactionnel est chauffé au reflux (38,5°C sous une pression de 410 mm de mercure; 54,3 kPa). On fait alors passer un courant de chlore gazeux pendant 29 minutes. La quantité totale de chlore introduite est de 7,9 g (111 m.moles). En même temps que l'on fait passer le chlore, on distille le méthyltertiobutyléther tout en maintenant constant le volume réactionnel par addition continue de méthyltertiobutyléther. La quantité totale de méthyltertiobutyléther distillé est égale à 470 cm³.

On opère ensuite comme dans l'exemple 1.

Par chromatographie gaz-liquide, on dose:

- triméthyl-2,4,6 phénol    12,9 m.moles
- hydroxy-4 triméthyl-2,4,6 cyclohexadiène-2,5 one    64,1 m.moles

Le taux de transformatiom du triméthyl-2,4,6 phénol est de 87,1 %.

Le rendement en hydroxy-4 triméthyl-2,4,6 cyclohexadiène-2,5 one est de 73,5 %.

**Revendications**

1. Procédé de préparation de l'hydroxy-4 triméthyl-2,4,6 cyclohexadiène-2,5 one par chloration du triméthyl-2,4,6 phénol au moyen de chlore dans un solvant organique choisi parmi les hydrocarbures aliphatiques ou cycloaliphatiques, les hydrocarbures aliphatiques polychlorés, les hydrocarbures aromatiques, les hydrocarbures aromatiques chlorés, les éthers, les esters et les nitriles, suivie de l'hydrolyse du mélange réactionnel caractérisé en ce que, lors de la chloration, effectuée en l'absence de base, l'acide chlorhydrique formé est éliminé au fur et à mesure de sa formation par tout moyen physique approprié.

2. Procédé selon la revendication 1 caractérisé en ce que l'acide chlorhydrique gazeux formé est éliminé par passage d'un courant de gaz inerte.

3. Procédé selon la revendication 1 caractérisé en ce que l'acide chlorhydrique gazeux formé est éliminé par distillation du solvant éventuellement sous pression réduite.

4. Procédé selon la revendication 3 caractérisé en ce que le volume du mélange réactionnel est maintenu constant par addition en continu d'une quantité de solvant propre égale à la quantité de solvant distillé.

5. Procédé selon la revendication 3 caractérisé en ce que la quantité de solvant distillé est remplacée partiellement par addition en continu de solvant propre.

6. Procédé selon la revendication 3 caractérisé en ce que la quantité de solvant distillé n'est pas remplacée par addition de solvant propre.

7. Procédé selon la revendication 1 caractérisé en ce que la concentration en triméthyl-2,4,6 phénol dans le solvant est comprise entre 0,1 mole par litre et la saturation.

8 - Procédé selon la revendication 1 caractérisé en ce que la chloration est effectuée à une température comprise entre -20 et 100°C.

**Patentansprüche**

1. Verfahren zur Herstellung von 4-Hydroxy-2,4,6-trimethyl-2,5-cyclohexadien-on durch Chlorieren von 2,4,6-Trimethylphenol mittels Chlor in einem organischen Lösungsmittel, ausgewählt aus den aliphatischen oder cycloaliphatischen Kohlenwasserstoffen, den polychlorierten aliphatischen Kohlenwasserstoffen, den aromatischen Kohlenwasserstoffen, den chlorierten aromatischen Kohlenwasserstoffen, den Äthern, den Estern und den Nitrilen, und anschließende Hydrolyse der Reaktionsmischung, dadurch gekennzeichnet, daß bei der Chlorierung, die in Abwesenheit einer Base ausgeführt wird, die entstehende Salzsäure gleichzeitig durch eine geeignete physikalische Maßnahme entfernt wird.

2. Verfahren nach Anspruch 1, dadurch gekennzeichnet, daß die gebildete gasförmige Salzsäure durch Durchleiten eines inerten Gasstromes entfernt wird.

3. Verfahren nach Anspruch 1, dadurch gekennzeichnet, daß die gebildete gasförmige Salzsäure durch Destillation des Lösungsmittels, gegebenenfalls unter vermindertem Druck, entfernt wird.

4. Verfahren nach Anspruch 3, dadurch gekennzeichnet, daß das Volumen der Reaktionsmischung durch kontinuierliche Zugabe einer Menge des entsprechenden Lösungsmittels, die der Menge des destillierten Lösungsmittels entspricht, konstant gehalten wird.

5. Verfahren nach Anspruch 3, dadurch gekennzeichnet, daß die destillierte Lösungsmittelmenge teilweise durch kontinuierliche Zugabe des entsprechenden Lösungsmittels ersetzt wird.

6. Verfahren nach Anspruch 3, dadurch gekennzeichnet, daß die destillierte Lösungsmittelmenge nicht durch Zugabe des entsprechenden Lösungsmittels ersetzt wird.

7. Verfahren nach Anspruch 1, dadurch gekennzeichnet, daß die Konzentration des 2,4,6-Trimethylphenols im Lösungsmittel zwischen 0,1 Mol pro Liter und der Sättigung liegt.

8. Verfahren nach Anspruch 1, dadurch gekennzeichnet, daß die Chlorierung bei einer Temperatur zwischen -20 und 100° C ausgeführt wird.

## Claims

Process for the preparation of 4-hydroxy-2,4,6-trimethyl-2,5-cyclohexadienone by chlorination of 2,4,6-trimethylphenol by means of chlorine in an organic solvent chosen from aliphatic or alicyclic hydrocarbons, polychlorinated aliphatic hydrocarbons, aromatic hydrocarbons, chlorinated aromatic hydrocarbons, ethers, esters and nitriles, followed by hydrolysis of the reaction mixture, characterized in that, during the chlorination, which is performed in the absence of base, the hydrochloric acid formed is removed as it is formed by any suitable physical means.

2. Process according to Claim 1, characterized in that the gaseous hydrochloric acid formed is removed by passing a stream of inert gas.

3. Process according to Claim 1, characterized in that the gaseous hydrochloric acid formed is removed by distillation of the solvent, under reduced pressure if desired.

4. Process according to Claim 3, characterized in that the volume of the reaction mixture is kept constant by continuous addition of a quantity of clean solvent which is equal to the quantity of distilled solvent.

5. Process according to Claim 3, characterized in that the quantity of distilled solvent is partly replaced by continuous addition of clean solvent.

6. Process according to Claim 3, characterized in that the quantity of distilled solvent is not replaced by adding clean solvent.

7. Process according to Claim 1, characterized in that the concentration of 2,4,6-trimethylphenol in the solvent is between 0.7 mole per litre and saturation.

8. Process according to Claim 1, characterized in that the chlorination is carried out at a temperature between -20 and 100° C.